# EUROPEAN PATENT APPLICATION

(11) **EP 4 296 989 A1**
(43) Date of publication of application: **27.12.2023**
(21) Application number: 21912332.0
(22) Date of filing: 18.02.2021
(51) Int. Cl.: G08G 1/16

(54) **INFORMATION PROCESSING DEVICE, OUTPUT CONTROL METHOD, AND OUTPUT CONTROL PROGRAM**

(71) Applicant: Pioneer Corporation, Tokyo 113-0021 (JP)
(72) Inventor: SUZUKI, Yasunori, Kawagoe-shi, Saitama 350-8555 (JP); SOMA, Takayuki, Kawagoe-shi, Saitama 350-8555 (JP); MURATA, Kazuo, Kawagoe-shi, Saitama 350-8555 (JP); KASHIO, Shota, Kawagoe-shi, Saitama 350-8555 (JP)
(74) Representative: Hoffmann Eitle
(86) International application number: PCT/JP2021/006228
(87) International publication number: WO 2022/176136

(57) **Abstract**

An information processing apparatus (1) includes: an identifying unit (11) that identifies a place where a driver of a vehicle is under a duty of care; an obtaining unit (12) that obtains travel information on the vehicle at the place identified by the identifying unit (11); an accumulating unit (13) that accumulates the travel information on the vehicle, the travel information having been obtained by the obtaining unit (12); and an output control unit (17) that outputs information related to fatigue in the driver, on the basis of the travel information on the vehicle, the travel information having been accumulated by the accumulating unit (13).

## Description

### Field

The present invention relates to an information processing apparatus, an output control method, and an output control program.

### Background

For detection of any abnormality in a driver, information, such as a trip or sitting time period, a driving time period, or a stress hormone index, is used. Furthermore, an information providing apparatus described in Patent Literature 1 uses a level of instability calculated from an index based on steering operation and pedaling operation, an index based on frequency of lever operation, and an index based on acceleration/deceleration (see, for example, Patent Literature 1).

### Citation List

### Patent Literature

Patent Literature 1: Japanese Unexamined Patent Application Publication No. 2011-204120

### Summary

### Technical Problem

However, conventional techniques including the technique described in Patent Literature 1, in one aspect, do not enable determination of whether an abnormality detected comes from stress or fatigue. The conventional technique mentioned above thus has a problem that information related to fatigue is reduced in accuracy. This problem is one of examples of problems to be solved by the present invention.

The present invention has been made in view of the above, and an object thereof is to provide an information processing apparatus, an output control method, and an output control program that enable improvement in accuracy of information related to fatigue, for example.

### Solution to Problem

An information processing apparatus described in claim 1 includes: an identifying means that identifies places where a driver of a vehicle is under a duty of care; an obtaining means that obtains travel information on the vehicle at the places identified by the identifying means; an accumulating means that accumulates travel information on the vehicle, the travel information having been obtained by the obtaining means; and an output control means that outputs information related to fatigue in the driver, on the basis of the travel information on the vehicle accumulated by the accumulating means.

An output control method described in claim 10 is implemented by an information processing apparatus which executes a process including: identifying a place where a driver of a vehicle is under a duty of care; obtaining travel information on the vehicle at the place identified; accumulating the obtained travel information on the vehicle; and outputting information related to fatigue in the driver, on the basis of the accumulated travel information on the vehicle.

An output control program described in claim 11 causes a computer to execute a process including: identifying a place where a driver of a vehicle is under a duty of care; obtaining travel information on the vehicle at the place identified; accumulating the obtained travel information on the vehicle; and outputting information related to fatigue in the driver, on the basis of the accumulated travel information on the vehicle.

### Brief Description of Drawings

FIG. 1 is a block diagram illustrating an example of a functional configuration of an information processing apparatus according to an embodiment.
FIG. 2 is a diagram illustrating an example of output of information related to fatigue.
FIG. 3 is a schematic diagram illustrating an example of a stop area.
FIG. 4 is a schematic diagram illustrating an example of a method of obtaining travel information.
FIG. 5 is a diagram illustrating an example of a first approximate straight line and a second approximate straight line.
FIG. 6 is a flowchart illustrating a procedure of an output control process according to the embodiment.
FIG. 7 is a schematic diagram illustrating an application example of an area to be under a duty of care.
FIG. 8 is a schematic diagram illustrating an application example of an area to be under a duty of care.
FIG. 9 is a schematic diagram illustrating an application example of an area to be under a duty of care.
FIG. 10 is a schematic diagram illustrating an application example of an area to be under a duty of care.
FIG. 11 is a schematic diagram illustrating an application example of an area to be under a duty of care.
FIG. 12 is a diagram illustrating an example of a functional configuration of a client server system according to an application example.
FIG. 13 is a diagram for explanation of an example of a hardware configuration.

### Description of Embodiments

Modes for implementing the present invention (hereinafter, embodiments) will hereinafter be described while reference is made to the drawings. The present invention is not limited by the embodiments described hereinafter. Furthermore, any portions that are the same will be assigned with the same reference sign, throughout the drawings.

### Example of Usage

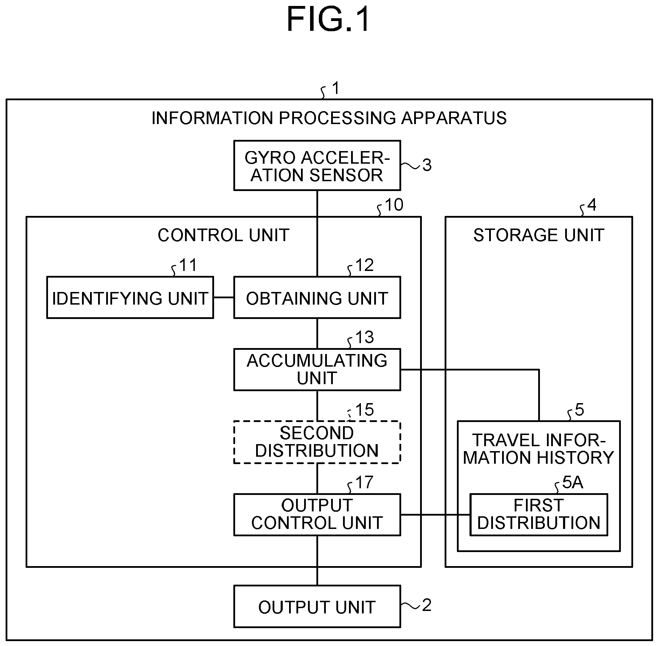

FIG. 1 is a block diagram illustrating an example of a functional configuration of an information processing apparatus according to an embodiment. An information processing apparatus 1 illustrated in FIG. 1 may be implemented by any computer, such as a smartphone, a tablet terminal, or a wearable terminal, which may be, by way of example only, a drive recorder, a navigation device, or an audio-visual (AV) device.

The information processing apparatus 1, in one aspect, may be used in a state where the information processing apparatus 1 has been brought into a vehicle. This is merely an example, does not mean that the usage is limited just to vehicle installation, and the information processing apparatus 1 may be used in a state where the information processing apparatus 1 has not been installed in a vehicle. A state where the information processing apparatus 1 has been brought into a vehicle may hereinafter be referred to as an "in-vehicle state".

### Example of Function Provided

The information processing apparatus 1 is capable of providing an output control function for executing control of output of information related to fatigue. An example where the output control function is provided in the in-vehicle state will be described hereinafter by way of example only, but the output control function and any other function or service may be provided in a state where the information processing apparatus 1 is in the in-vehicle state or any other state.

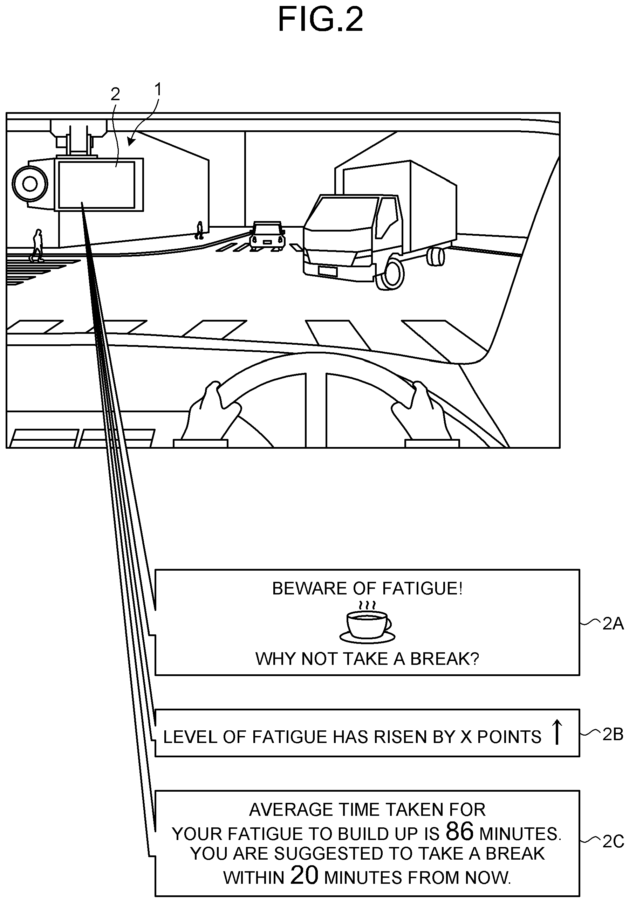

FIG. 2 is a diagram illustrating an example of output of information related to fatigue. FIG. 2 illustrates notifications 2A to 2C that are an example of information output to an output unit 2 by the output control function provided by the information processing apparatus 1 in the in-vehicle state.

As indicated by the notification 2A in FIG. 2, the output control function is capable of causing the output unit 2 to display an alert including a message, "Beware of fatigue! Why not take a break?" and an icon prompting to take a break, at a time of occurrence of an event, such as an increase in fatigue.

Without being limited to the time of occurrence of an event, the output control function may implement constant output or regular output of information related to fatigue, as exemplified by the notification 2B and the notification 2C in FIG. 2.

For example, as indicated by the notification 2B in FIG. 2, the output control function may cause the output unit 2 to display a message, "Level of fatigue has risen by X points", for notification of the degree of increase in fatigue in a driver, for example.

Furthermore, as indicated by the notification 2C in FIG. 2, the output control function may cause the output unit 2 to display a message, "Average time taken for your fatigue to build up is 86 minutes. You are suggested to take a break within 20 minutes from now.", for example. Notification of a statistical value of times each taken from start of the engine to output of an alert for increase in fatigue or a lead time prior to output of an alert may be performed as described above.

Processing and operation in a case where a notification A of the notification 2A to notification 2C is executed by the above described output control function will hereinafter be described by way of example only, but the embodiment is not limited to this example. That is, it is additionally mentioned herein beforehand that the above described output control function enables execution of at least any one of the notification 2A, the notification 2B, the notification 2C, any other notification, or any combination of these.

### Example of Problem

As described in the above section, Technical Problem, the above mentioned conventional technique, in one aspect, does not enable determination of whether an abnormality detected comes from stress or fatigue. The above mentioned conventional technique thus has a problem that information related to fatigue is reduced in accuracy. Accordingly, the above mentioned problem is one of examples of problems to be solved by the output control function according to the embodiment.

### Example of Problem Solving Approach

For the output control function according to the embodiment, a problem solving approach to narrow travel information used in evaluation of a degree of buildup of fatigue down to travel information on places where a driver is under a duty of care is adopted. A place where a driver is under a duty of care may hereinafter be referred to as an "area to be under a duty of care".

Motivation to adopt such a problem solving approach is only obtained after the following technical findings are obtained.

A driver is at fault for a discontinuous driving behavior at a stop as indicated by the following (A) to (C). The "discontinuous driving behavior" referred to herein, in one aspect, refers to non-smooth operation.
(A) If the driver is capable of adequate risk prediction, the driver would be driving at the driver's own pace and driving comparatively smoothly even when approaching to stop.
(B) A discontinuous driving behavior is thought to be caused by an unexpected disturbance that was unable to be predicted or by an error in the driver's risk prediction.
(C) When a vehicle on a non-priority road enters a priority road, stopping is a traffic rule (regulation) to be fulfilled without fail and thus if the driver brakes suddenly, the driver is more at fault than the other.

Examples of disadvantages when areas from which a degree of buildup of fatigue is evaluated are not narrowed down to areas to be under a duty of care include the following (D) to (J) .
(D) Determination of whether a driver is at fault or the other is at fault is not possible.
(E) If the other is at fault, the driver will have a feeling of anger rather than fatigue.
(F) If the driver's vehicle is traveling in a straight line, the driver will always be at a place where the other at fault is in front or back of the driver.
(G) This tends to lead to tailgating (excessive stress relief).
(H) A mere stop at a light, for example, is also one of causes of a discontinuous driving behavior.
(I) Being discontinuous does not necessarily lead to stress or fatigue.
(J) In terms of "physical fatigue + mental fatigue", evaluation should be done on the basis of what the driver is at fault for.

As indicated by (D) to (J) above, in a case where evaluation of a degree of buildup of fatigue is not limited to areas to be under a duty of care, disturbances, stress, and feelings that are not dependent on the driver's responsibility, for example, will be confused with fatigue in evaluation.

To corroborate (D) to (J) above, comparison with case examples where discontinuous driving behaviors occur in places other than areas to be under a duty of care will be made.

A first case example is where "The other has cut in aggressively ahead of the driver!". The driver feels stress but this stress is not likely to lead to fatigue. This stress can rather be said to be stress leading to anger and is likely to develop into excessive stress relief, such as tailgating.

A second case example is where "The other vehicle comes down in the wrong direction while the driver is traveling on a one-way street!". This case also leads to a discontinuous driving behavior, such as sudden braking, but this discontinuous driving behavior does not necessarily lead to buildup of fatigue. That is, the driver may be frightened for a moment or feel stress for a moment but this does not necessarily lead to fatigue. Furthermore, it does not necessarily mean that buildup of fatigue in the driver resulted in the discontinuous driving behavior, the sudden braking.

From these two case examples, it can be said that even a discontinuous driving behavior, such as sudden braking, does not necessarily lead to fatigue, the discontinuous driving behavior being for avoidance of potential accident cases and accidents, for example, in a case where the other driver is at fault and the other driver has neglected the other driver's duty of care. In addition, there still remains a possibility that the driver inevitably braked suddenly to avoid risk, instead of braking suddenly as a result of reduction in alertness due to fatigue in the driver.

From the above, the following technical findings (K) to (M) are obtained.
(K) A discontinuous driving behavior exerts stress and fatigue on the driver and stress and fatigue are often confused with each other.
(L) Fatigue (physical fatigue + mental fatigue) tends to build up all the more in places where the driver is under a duty of care.
(M) A degree of buildup of fatigue is levelled by focusing on driving behaviors in stop areas.

In a case where evaluation of a degree of buildup of fatigue is limited to areas to be under a duty of care on the basis of the technical findings (K) to (M), the degree of buildup of fatigue is able to be evaluated by distinction from disturbances and stress not dependent on the driver's responsibility as indicated by the following (N) to (P).
(N) A discontinuous driving behavior observed in an area to be under a duty of care is likely to be due to a disturbance or misreading in risk prediction and not caused by the driver's own will based on risk prediction.
(O) Even if the other driver is at fault, the driver is at fault because the driver entered a priority road from a non-priority road.
(P) Frequently encountering areas to be under a duty of care leads to buildup of stress and fatigue leading to fatigue.

Therefore, the output control function according to the embodiment enables improvement in accuracy of information related to fatigue.

### Configuration of Information Processing Apparatus 1

An example of a functional configuration of the information processing apparatus 1 according to the embodiment will be described next. FIG. 1 is a schematic of blocks corresponding to functions that the information processing apparatus 1 has. As illustrated in FIG. 1, the information processing apparatus 1 has an output unit 2, a gyro acceleration sensor 3, a storage unit 4, and a control unit 10. FIG. 1 merely illustrates excerpts corresponding to functional units related to the output control function described above, and the information processing apparatus 1 may include any other functional unit not illustrated in FIG. 1, for example, any functional unit to be included in an existing computer by default or optionally, such as a communication control unit.

The output unit 2 is a functional unit that outputs various kinds of information. By way of example only, the output unit 2 may be implemented by any of various display devices, such as, for example, a liquid crystal display, or an organic electroluminescence (EL) display. Furthermore, the output unit 2 may be implemented by, for example, a touch panel serving as a display input unit integrated with an input unit not illustrated in the drawings. Displaying may be implemented by projection, without being limited to a case where displaying is implemented by emission of light. In another example, the output unit 2 may be implemented by any of various sound output devices, for example, a speaker.

The gyro acceleration sensor 3 corresponds to an example of: an acceleration detecting unit; and an angular velocity detecting unit. A geomagnetic sensor may be used as another example of the angular detecting unit. For example, the gyro acceleration sensor 3 is capable of detecting, for example, acceleration along three axes, for example, an X-axis, a Y-axis, and a Z-axis, and angular velocity around three axes, for example, for rolling, pitching, and yawing. The example where acceleration along three axes and angular velocity around three axes are detected has been mentioned herein, but the numbers of axes for which acceleration and angular velocity are detected are not limited to three. Furthermore, the example where both acceleration and angular velocity are detected is described herein, but only one of these two may be detected.

The storage unit 4 is a functional unit to store various kinds of data. By way of example only, the storage unit 4 may be implemented by an internal, external, or auxiliary storage of the information processing apparatus 1 or a part of a storage area of that storage.

For example, every time a predetermined number of pieces, for example, 50 pieces, of travel information obtained by an obtaining unit 12 described later for respective areas to be under a duty of care have been accumulated in the storage unit 4, the predetermined number of pieces of the travel information are packaged as a travel information group, and this travel information group is additionally stored in the storage unit 4. Such addition is repeated and as a result, a set of travel information groups each having a package of the predetermined number of pieces of travel information is stored as a travel information history 5 into storage unit 4.

By way of example only, the travel information history 5 will be described below, the travel information history 5 having travel information that starts to be obtained by the obtaining unit 12 described later from start of an engine of a vehicle and that is accumulated up to stop of the engine. This is just an example, and travel information may continue to be accumulated until resetting is executed by a user setting or a system setting, needless to say.

The travel information may include time-series data on acceleration in traveling directions measured in areas to be under a duty of care or variance values of acceleration determined from the time-series data on acceleration in the traveling directions. Furthermore, the travel information may include time-series data on velocity in the traveling directions measured in the areas to be under a duty of care or average velocities determined from the time-series data on the velocity in the traveling directions.

Description will hereinafter be continued with respect to an example of the travel information, the example being driving behavior data represented by pairs of: variance values of acceleration in traveling directions obtained in areas to be under a duty of care; and average velocities in the traveling directions obtained in the areas to be under a duty of care. Accordingly, every time a predetermined number of pieces, for example, 50 pieces, of driving behavior data obtained for respective areas to be under a duty of care are accumulated in the storage unit 4, the predetermined number of pieces of driving behavior data are packaged as a driving behavior data group, and the driving behavior data group is additionally stored in the storage unit 4. In other words, the travel information history 5 is thus a set of driving behavior data groups each being a package of the predetermined number of pieces of driving behavior data.

Of the travel information history 5, a travel information group, that is, a driving behavior data group that is accumulated first since a time point at which the engine of a vehicle is started, in one aspect, may be regarded as being relatively low in fatigue of the driver as compared with that after start of travel of the vehicle. The driving behavior data group accumulated first since the time point at which the engine of the vehicle is started is distinguished as a first distribution 5A of driving behavior data, from other driving behavior data groups. This is to use the first distribution 5A as a target to be compared with a second distribution of driving behavior data for evaluating the increasing trend of the second distribution, the driving behavior data being a driving behavior data group obtained thereafter.

Information stored in the storage unit 4 is not limited to the travel information history 5. Other data may be stored in the storage unit 4, of course. For example, information used by the above described output control function, such as a degree of increase in fatigue, changes in the degree of increase, and a statistical value of times taken for fatigue to increase, may be stored in the storage unit 4. In addition, map data used in navigation of the vehicle and image data captured for recording, for example, may be stored in the storage unit 4.

The control unit 10 is a processing unit that executes overall control of the information processing apparatus 1. As illustrated in FIG. 1, the control unit 10 has an identifying unit 11, the obtaining unit 12, an accumulating unit 13, and an output control unit 17.

The identifying unit 11 is a processing unit that identifies a place where a driver of a vehicle is under a duty of care. For example, the identifying unit 11 corresponds to an example of an identifying means. By way of example only, the identifying unit 11 executes map matching between: positional information on the information processing apparatus 1, the positional information being measured by a positional information measuring unit not illustrated in the drawings; and areas to be under a duty of care that have been set on map data stored in the storage unit 4. This positional information measuring unit may be implemented by a global positioning system (GPS) receiver, for example.

For example, the areas to be under a duty of care may be set by use of information on road signs included in the map data. An example where areas to be under a duty of care in which a duty to stop is imposed on drivers of vehicles are set will hereinafter be described by way of example only. In this case, areas to be under a duty of care are set on the basis of structural change points on roads, such as junctions and transport nodes, including nodes where non-priority road signs of road signs are present, for example, intersections.

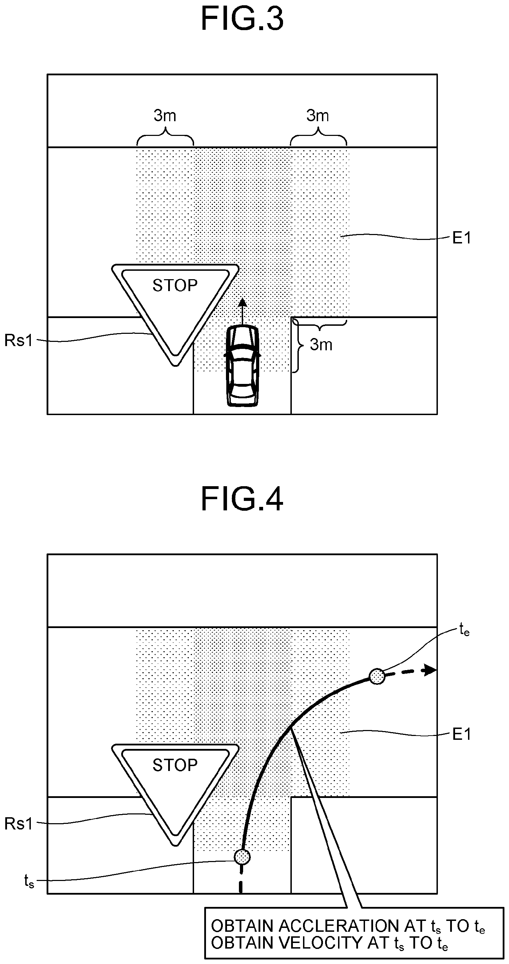

FIG. 3 is a schematic diagram illustrating an example of a stop area. FIG. 3 illustrates an example where a stop area E1 is set at an intersection of a T-junction having a non-priority road sign Rs1. As illustrated in FIG. 3, in the stop area E1, an intersection (a dark hatched portion) and a range (light hatched portions) within a predetermined distance, for example, three meters, from a boundary of the intersection are set. FIG. 3 illustrates the example where a stop area is set at an intersection of a T-junction, but a stop area may be similarly set at an intersection other than that of a T-junction, needless to say.

With such stop areas having been set, the identifying unit 11 identifies passage through a stop area by executing map matching between positional information on the information processing apparatus 1 and the stop areas. In a case where passage through a stop area has been identified, the identifying unit 11 further determines whether or not the direction of passage through the stop area corresponds to travel from a non-priority road to a priority road.

The obtaining unit 12 is a processing unit that obtains travel information on a vehicle in a stop area. For example, the obtaining unit 12 corresponds to an example of an obtaining means. By way of example only, in a case where the direction of passage through a stop area corresponds to travel from a non-priority road to a priority road, the obtaining unit 12 obtains travel information for the stop area.

FIG. 4 is a schematic diagram illustrating an example of a method of obtaining travel information. FIG. 4 illustrates, as an example: the stop area E1 illustrated in FIG. 3 by use of dark hatching and light hatching; and the trajectory of positional information on a vehicle by use of an arrow in the direction corresponding to elapse of time. Furthermore, FIG. 4 illustrates: a portion of the line of the arrow by use of a solid line, the portion being included in the stop area E1; and portions of the line by use of broken lines, the portions not being included in the stop area E1. As illustrated in FIG. 4, the obtaining unit 12 identifies a time tₛ at which entrance into the stop area E1 started and a time tₑ at which departure from the stop area E1 ended. Thereafter, the obtaining unit 12 obtains time-series data on triaxial acceleration corresponding to an interval between the time tₛ and the time tₑ, from a log of the gyro acceleration sensor 3, for example.

After acceleration has been obtained as described above, the obtaining unit 12 executes the following processing for all of triaxial acceleration in the time-series data on triaxial acceleration, the all of triaxial acceleration corresponding to sampling frequencies of the gyro acceleration sensor 3, or for each value of triaxial acceleration resampled at predetermined intervals, for example, at one-second intervals.

That is, the obtaining unit 12 analyzes the traveling direction from triaxial acceleration that is acceleration along the X-axis, acceleration along the Y-axis, and acceleration along the Z-axis. Specifically, the obtaining unit 12 removes gravitational acceleration from composite acceleration that is a composite of triaxial acceleration. The obtaining unit 12 then projects a vector of the composite acceleration from which the gravitational acceleration has been removed, onto a horizontal plane. For example, the horizontal plane may be calculated beforehand through calibration by use of triaxial acceleration at a time when the information processing apparatus 1 is stationary, for example, immediately after starting. The obtaining unit 12 is then able to analyze the traveling direction from the vector of the composite acceleration projected on the horizontal plane.

Such analysis of the traveling direction enables obtainment of time-series data on acceleration in the traveling directions measured in the stop area. Furthermore, time-series data on acceleration in the traveling directions is obtained by integration of the time-series data on the acceleration in the traveling directions. The example where velocity is calculated by integration of acceleration has been described herein but velocity may be obtained by another method. For example, velocity may be obtained by obtainment of vehicle velocity pulses from a vehicle velocity signal line of a vehicle or by obtainment of a vehicle velocity signal via an electronic control unit (ECU) installed in a vehicle by use of on-board diagnostics (OBD2) functions.

By calculating variance from the time-series data on acceleration, the obtaining unit 12 obtains a variance value of acceleration. Furthermore, the obtaining unit 12 obtains an average velocity by calculating an average from the time-series data on velocity. Accordingly, driving behavior data represented by a pair of: the variance value of acceleration in the traveling direction obtained in an area to be under a duty of care; and the average velocity in the traveling direction obtained in the area to be under a duty of care is thus obtained.

The accumulating unit 13 is a processing unit that accumulates travel information on a vehicle, the travel information having been obtained by the obtaining unit 12. For example, the accumulating unit 13 corresponds to an example of an accumulating means. By way of example only, the accumulating unit 13 repeats the following processing after the engine of the vehicle is started until the engine is stopped. For example, start and stop of the engine may be detected by determination of: presence or absence of power feeding corresponding to insertion or removal into or from an accessory socket or a cigar socket in the vehicle; or a prolonged stop, for example, a stop for one hour or longer. After start of the engine is detected, every time driving behavior data for a stop area is obtained by the obtaining unit 12, the accumulating unit 13 accumulates the driving behavior data in a predetermined storage area on a memory. As the number of pieces of driving behavior data on stop areas reaches a predetermined number, for example, 50, a second distribution 15 of the driving behavior data is obtained. The second distribution 15 of the driving behavior data is additionally saved in the travel information history 5 stored in the storage unit 4 after being referred to by the output control unit 17 described later.

The output control unit 17 is a processing unit that outputs, on the basis of the travel information on the vehicle accumulated by the accumulating unit 13, information related to fatigue in the driver. For example, the output control unit 17 corresponds to an example of an output control means.

By way of example only, the output control unit 17 controls whether or not to output information related to fatigue in a driver, on the basis of an increasing trend of a second approximate straight line approximating the second distribution 15 of the driving behavior data accumulated by the accumulating unit 13, in relation to a first approximate straight line approximating the first distribution 5A of the driving behavior data stored in the storage unit 4.

For example, the first approximate straight line and the second approximate straight line are able to be calculated by execution of regression analysis, such as multiple regression, of the first distribution 5A of the driving behavior data or the second distribution 15 of the driving behavior data. A partial regression coefficient of the first approximate straight line may be stored in a memory or the storage unit 4 after being calculated in a first round, and calculation of multiple regression in a second round and later rounds may be skipped.

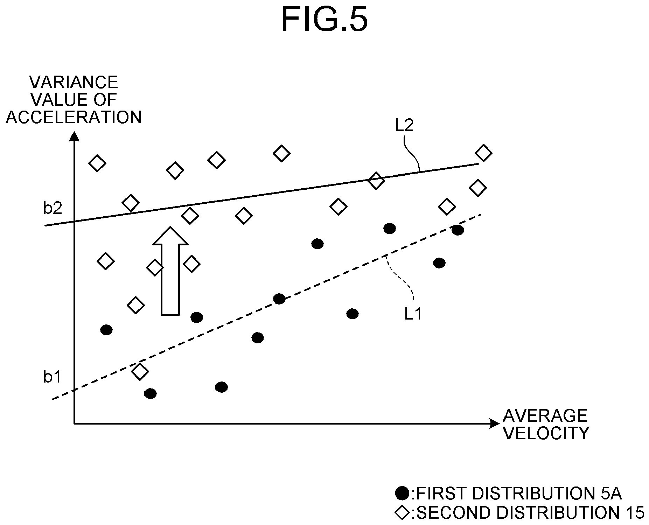

FIG. 5 is a diagram illustrating an example of the first approximate straight line and the second approximate straight line. FIG. 5 illustrates a graph having the variance of acceleration along the vertical axis and the average velocity along the horizontal axis. Furthermore, in FIG. 5, while a driving behavior data group corresponding to the first distribution 5A is plotted with circles, a driving behavior data group corresponding to the second distribution 15 is plotted with rhombuses. In addition, while a first approximate straight line L1 approximating the first distribution 5A of the driving behavior data is represented by a broken line, a second approximate straight line L2 approximating the second distribution 15 of the driving behavior data is represented by a solid line.

As illustrated in FIG. 5, the larger the velocity, the larger the degree of sudden braking upon braking and the variance value, and the first approximate straight line L1 is thus approximated by an upward sloping straight line. The second approximate straight line L2 is also an upward sloping graph. However, the range of variance values of acceleration on the second approximate straight line L2 is narrower than the range of variance values of acceleration on the first approximate straight line L1. Furthermore, there is a significant gap in variance values of acceleration observed between the first approximate straight line L1 and the second approximate straight line L2 when the average velocity is in a low velocity range. That is, while not many discontinuous driving behaviors are observed in a low velocity range for the first approximate straight line L1, discontinuous driving behaviors tend to be observed in the low velocity range for the second approximate straight line L2. This indicates that: when the average velocity is high (the scale of the intersection is large), the driver is placed under control by the traffic light and the variance values are thus just a little higher for L2 than L1 in the range where the average velocity is high even if fatigue is being built up; and the risk of a child or a bicycle running into the street is higher when the average velocity is low (the scale of the intersection is small), and thus the lower the velocity is, the more affected the level of buildup of fatigue. Accordingly, comparing an intercept b1 of the first approximate straight line L1 with an intercept b2 of the second approximate straight line L2 is evidently effective for determination of the increasing trend of the second approximate straight line L2 in relation to the first approximate straight line L1, that is, the degree of buildup of fatigue.

Therefore, the output control unit 17 determines whether or not the intercept b2 of the second approximate straight line L2 is equal to or larger than a threshold Th1 set on the basis of the intercept b1 of the first approximate straight line L1. A value larger than the intercept b1, for example, the intercept b1 + a margin α, may be set as this threshold Th1. In a case where the intercept b2 of the second approximate straight line L2 is equal to or larger than the threshold Th1, the degree of buildup of fatigue is able to be presumed to have increased. In this case, the output control unit 17 outputs information related to fatigue in the driver. For example, as exemplified by the notification 2A in FIG. 2, the output control unit 17 may cause the output unit 2 to display an alert including the message, "Beware of fatigue! Why not take a break?" and the icon prompting to take a break. The alert is not necessarily implemented by display output and may be implemented by sound output. For example, an alarm, such as a beep, may be sounded, or automated voice may be caused to read text corresponding to a message.

As described above, the output control unit 17 determines an increasing trend of a second approximate straight line in relation to a first approximate straight line. Therefore, in contrast to evaluation of the degree of buildup of fatigue by comparison between driving behavior data and a threshold statistically determined, the degree of buildup of fatigue is able to be evaluated by autocorrelation of driving behavior data. Therefore, even in a case where there are individual differences among degrees of buildup of fatigue in drivers, the degrees of buildup of fatigue are able to be evaluated appropriately. As a result, mental fatigue caused by announcements made to a driver who is not fatigued is able to be reduced, and omission of detection of a driver who is fatigued is able to be reduced.

FIG. 5 illustrates the example where the vertical axis of the graph corresponds to the variance value of acceleration, but the vertical axis may correspond to, instead of the variance value of acceleration, the degree of sudden braking, for example, deceleration, the degree being similar to the variance value of acceleration. Furthermore, FIG. 5 illustrates the example where the horizontal axis of the graph corresponds to the average velocity, but the horizontal axis may correspond to, instead of the average velocity, the median or mode of velocity, or the intersection scale, the median or mode and the intersection scale being similar to the average velocity. As to the "intersection scale" mentioned herein, scales of nodes of stop areas may be grouped by level from information on widths or the numbers of lanes associated with links connected to the nodes of the stop areas, the links being from links of a road network included in map data.

Furthermore, as one example of the threshold Th1, the example where the intercept b1 + margin α is used has been described herein, but the threshold Th1 is not limited to this example. For example, a distribution of driving behavior data at times of occurrence of accidents may be obtained, and the threshold Th1 may be set on the basis of this distribution. In addition, the threshold Th1 may be set on the basis of changes in the intercept obtained by calculation of an intercept of an approximate straight line for each driving behavior data group from a set of driving behavior data groups included in the travel information history 5.

### Flow of Processing

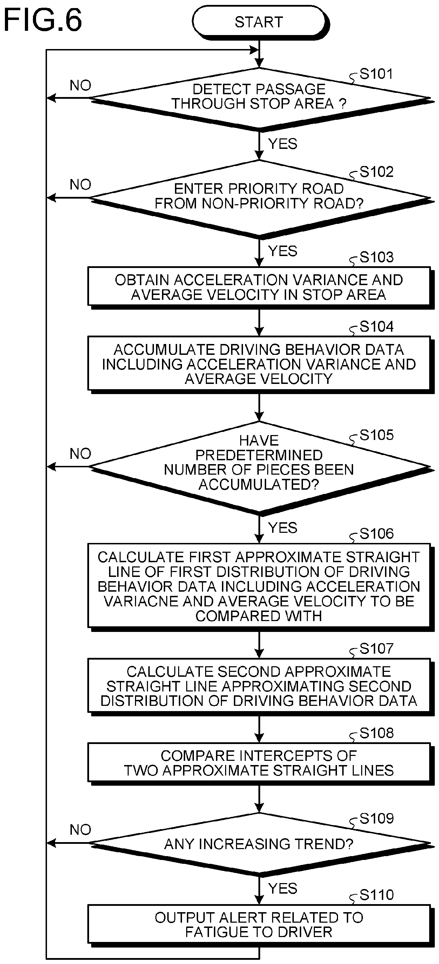

A flow of processing by the information processing apparatus 1 according to the embodiment will be described next. FIG. 6 is a flowchart illustrating a procedure of an output control process according to the embodiment. By way of example only, this process may be repeated from a time at which the power of the information processing apparatus 1 is turned on to a time at which the power is turned off, or from detection of start of an engine to detection of stop of the engine.

As illustrated in FIG. 6, the identifying unit 11 identifies passage through a stop area by executing map matching between positional information on the information processing apparatus 1 and stop areas (Step S101).

In a case where passage through a stop area has been identified (Yes at Step S101), the identifying unit 11 further determines whether or not the direction of the passage through the stop area corresponds to travel from a non-priority road to a priority road (Step S102).

In a case where the direction of the passage through the stop area corresponds to travel from a non-priority road to a priority road (Yes at Step S102), the obtaining unit 12 then executes the following processing. That is, the obtaining unit 12 obtains driving behavior data represented by a pair of a variance value of acceleration in the traveling direction obtained in the stop area and an average velocity in the traveling direction obtained in the area to be under a duty of care (Step S103). Subsequently, the accumulating unit 13 accumulates the driving behavior data for the stop area obtained at Step S103 into a predetermined storage area on a memory (Step S104).

In a case where the number of pieces of driving behavior data for stop areas accumulated in the memory has reached a predetermined number, for example, 50 (Yes at Step S105), the output control unit 17 then executes the following processing. That is, the output control unit 17 calculates the first approximate straight line approximating the first distribution 5A of the driving behavior data stored in the storage unit 4 (Step S106). For the distribution and the approximate line at S106, fixed values may be used from past histories or experimental data, for example.

Furthermore, the output control unit 17 calculates the second approximate straight line approximating the second distribution 15 of the driving behavior data accumulated by the accumulating unit 13 (Step S107). Subsequently, the output control unit 17 compares the intercept b1 of the first approximate straight line L1 with the intercept b2 of the second approximate straight line L2 (Step S108) .

In a case where the intercept b2 of the second approximate straight line L2 is equal to or larger than the threshold Th1 set on the basis of the intercept b1 of the first approximate straight line L1 (Yes at Step S109), the degree of buildup of fatigue is presumed to have increased. In this case, the output control unit 17 outputs information related to fatigue in the driver (Step S110) and proceeds to Step S101.

In a case where the processing is advanced to the branch of No at Step S101, No at Step S102, No at Step S105, or No at Step S109, the processing proceeds to Step S101.

### Examples of Effects of Embodiment

As described above, the information processing apparatus 1 according to the embodiment narrows travel information to be used in evaluation of a degree of buildup of fatigue down to travel information on places where the driver is under a duty of care. Evaluation of the degree of buildup of fatigue by distinction from disturbances and stress not dependent on the driver's responsibility is thereby enabled. Therefore, the information processing apparatus 1 according to the embodiment enables improvement in accuracy of information related to fatigue.

Furthermore, the information processing apparatus 1 of this embodiment controls, on the basis of an increasing trend of a second approximate straight line approximating a second distribution of driving behavior data in relation to a first approximate straight line approximating a first distribution of driving behavior data, whether or not information related to fatigue in the driver is to be output. Therefore, in contrast to evaluation of the degree of buildup of fatigue by comparison between driving behavior data and a threshold statistically determined, the degree of buildup of fatigue is able to be evaluated by autocorrelation of driving behavior data. Accordingly, even in a case where there are individual differences among degrees of buildup of fatigue in drivers, the information processing apparatus 1 of the embodiment enables reduction in: mental fatigue caused by provision of information to a driver who is not fatigued; and omission in provision of information to a driver who is fatigued.

Furthermore, on the basis of whether or not an intercept of a second approximate straight line is equal to or larger than a threshold determined by an intercept of a first approximate straight line, the information processing apparatus 1 of this embodiment controls whether or not information related to fatigue in a driver is to be output. Increase in the degree of buildup of fatigue is thereby able to be determined with respect to the intercept significantly representing the gap in the driving behaviors between the first approximate straight line and the second approximate straight line. Therefore, even for a driver who has a smaller gap between driving behaviors at the time when the driver is fatigued and the time when the driver is not fatigued than other drivers, the information processing apparatus 1 of the embodiment enables information related to the fatigue to be provided while enabling misdetection and omission in detection for increase in the degree of buildup of fatigue.

Furthermore, the information processing apparatus 1 of this embodiment selects a first distribution that is a distribution of driving behavior data accumulated first since a time point at which the engine of the vehicle is started. Therefore, the first approximate straight line representing relatively lower fatigue in the driver than that after start of travel of the vehicle is selected. The information processing apparatus 1 of this embodiment thus enables determination of increase in the degree of buildup of fatigue by comparison with the first approximate straight line in which the gap in the driving behavior from the second approximate straight line tends to appear.

Furthermore, the information processing apparatus 1 of this embodiment identifies a stop area, obtains travel information on the stop area, accumulates the obtained travel information on the stop area, and executes information related to fatigue in the driver on the basis of the accumulated travel information on the stop area. The degree of buildup of fatigue for when the driver is under a duty to stop is thereby able to be evaluated, the duty to stop being higher in percentage of negligence among duties of care the driver is under. Therefore, the information processing apparatus 1 according to the embodiment enables more effective improvement in accuracy of information related to fatigue.

Furthermore, the information processing apparatus 1 of this embodiment outputs information related to fatigue in a driver, the information being: the degree of increase in fatigue in the driver; changes in the degree of increase; an alert to the driver and related to the fatigue, and a statistical value of times each taken from a time point at which the engine of a vehicle is started to output of an alert. Therefore, the information processing apparatus 1 of this embodiment enables increase in the degree of buildup of fatigue to be announced in many aspects.

### Application Examples

The above described embodiment is one of examples, and various applications are possible.

### (1) Application Example of Method of Identifying Area to be Under Duty of Care

With respect to the above described embodiment, the example where passage through an area to be under a duty of care is identified by map matching has been described, but passage through an area to be under a duty of care may be identified by another method. For example, instead of map matching, passage through an area to be under a duty of care may be identified by image recognition for an image captured by an imaging unit installed in the information processing apparatus 1, or passage through an area to be under a duty of care may be identified by combination of map matching and image recognition.

### (2) Area to be Under Duty of Care Other Than Stop Area

With respect to the above described embodiment, stop areas have been mentioned as an example of areas to be under a duty of care, but without being limited to this example, the output control function may be applied to an area to be under a duty of care that is not a stop area.

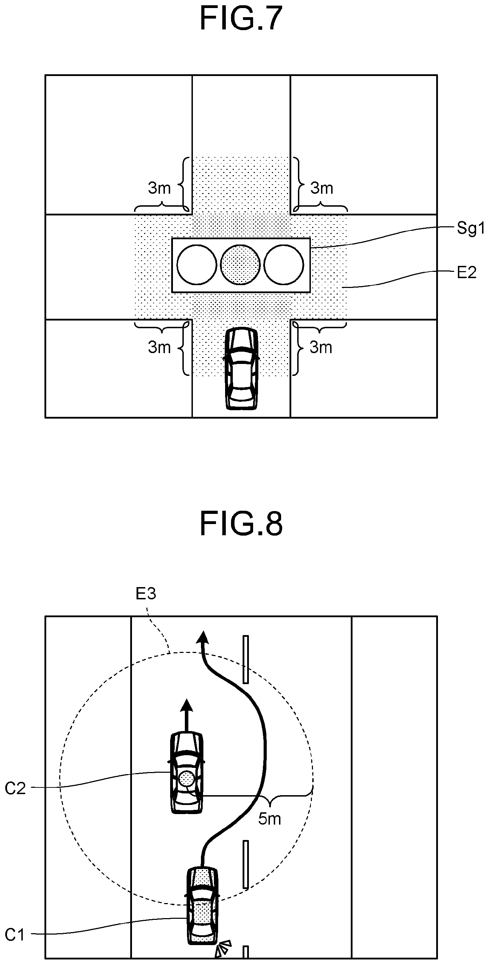

FIG. 7 is a schematic diagram illustrating an application example of an area to be under a duty of care. FIG. 7 illustrates an example where a flashing signal area E2 is set at an intersection of a cross-shaped junction where a traffic light Sg1 that displays a flashing signal is provided. Furthermore, in FIG. 7, the traffic light Sg1 having a flashing yellow light and set on a non-priority road has been excerpted for illustration from traffic lights provided at the intersection of the cross-shaped junction. As illustrated in FIG. 7, the intersection (a dark hatched portion) and a range within a predetermined distance, for example, 3 meters (dark hatched portions) from a boundary of the intersection are set in the flashing signal area E2, similarly to the case of the stop area E1 illustrated in FIG. 3. FIG. 7 illustrates the example where a flashing signal area is set at an intersection of a cross-shaped junction, but a flashing signal area may be similarly set at an intersection that is not of a cross-shaped junction.

FIG. 8 is a schematic diagram illustrating an application example of an area to be under a duty of care. FIG. 8 illustrates an area to beware of overtaking E3 that is set in a case where a vehicle C1 overtakes a vehicle C2 on a road having one lane each way and having a center line that is a white line, for example. In this case, if composition in which the vehicle C1 overtakes the vehicle C2 by going over the center line has been image-recognized, the area to beware of overtaking E3 is set by use of positional information on the vehicle C2. In the example illustrated in FIG. 8, a range within a predetermined distance, for example, five meters, from the positional information on the vehicle C2, that is, the inside of a broken-lined circle, is set as the area to beware of overtaking E3. In a case where travel information on the area to beware of overtaking E3 is to be obtained, travel information on an interval may be obtained, the interval being from the image recognition of the composition in which the vehicle C1 overtakes the vehicle C2 by going over the center line to image recognition of composition in which the vehicle C1 is traveling in front of the vehicle C2. Travel information on an interval from detection of operation of a direction indicator for a right turn to undoing of the operation of the direction indicator may be obtained instead. FIG. 8 illustrates the example where the center line is a white line, but the area to beware of overtaking E3 may be set similarly even if the center line is a broken line and/or a yellow line, needless to say.

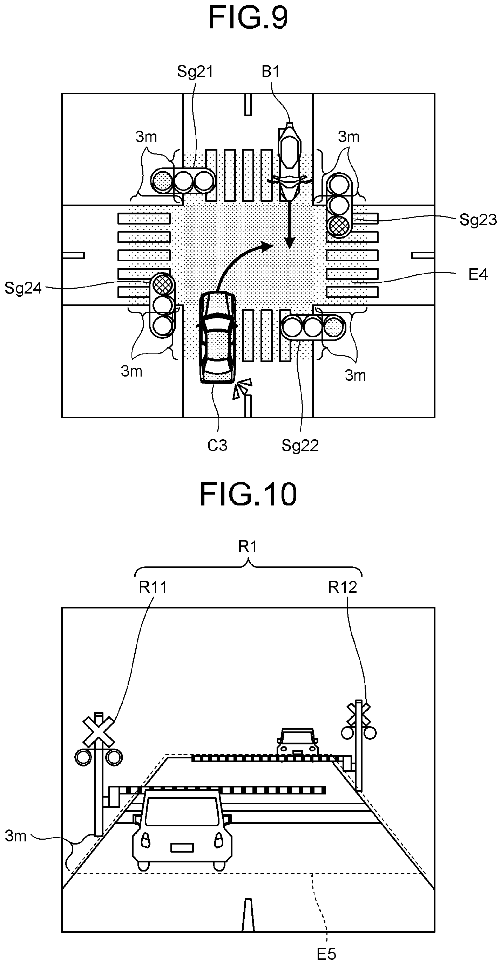

FIG. 9 is a schematic diagram illustrating an application example of an area to be under a duty of care. FIG. 9 illustrates, for example, an area to beware in turning right E4 set upon a right turn at an intersection of a cross-shaped junction where traffic lights Sg21 to Sg24 that display light signals have been provided. As illustrated in FIG. 9, the intersection (a dark hatched portion) and a range within a predetermined distance, for example, 3 meters (dark hatched portions), from a boundary of the intersection are set in the area to beware in turning right E4, similarly to the case of the stop area E1 illustrated in FIG. 3. In the area to beware in turning right E4, the driver of a vehicle C3 is under a duty of care for a vehicle going straight B1 on a green light of the traffic light Sg22, when the vehicle C3 on a green light of the traffic light Sg21 makes a right turn. Therefore, passage through the area to beware in turning right E4 is identified limitedly in a case where a direction indicator has been operated for a right turn or in a case where a right turning route has been set as a navigation route for the intersection. FIG. 9 illustrates the example where an area to beware in turning right is set at an intersection of a cross-shaped junction, but an area to beware in turning right may be similarly set at an intersection that is not of a cross-shaped junction.

FIG. 10 is a schematic diagram illustrating an application example of an area to be under a duty of care. FIG. 10 illustrates, for example, an area to beware of a railroad crossing E5 set on a road having one lane each way where a railroad crossing R1 including a crossing gate R11 and a crossing gate R12 has been provided. As illustrated in FIG. 10, in the area to beware of a railroad crossing E5, ranges of the crossing gate R11 and crossing gate R12, and a range within a predetermined distance, for example, three meters, toward this side, from the crossing gate R11 positioned on this side of the railroad crossing R1, are set. FIG. 10 illustrates the example where an area to beware of a railroad crossing is set on a road having one lane each way, but an area to beware of a railroad crossing may be similarly set on, for example, a two-way road where a railroad crossing has been provided, needless to say.

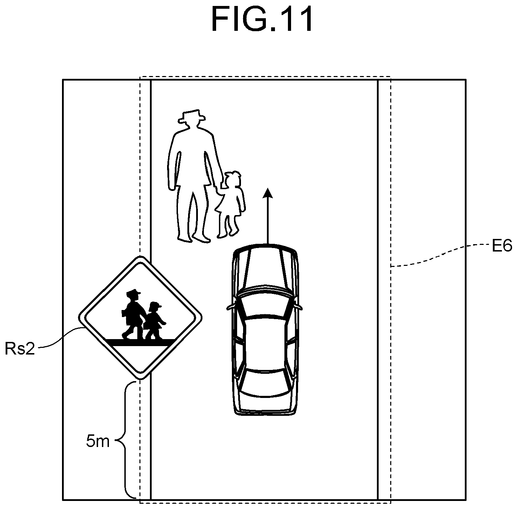

FIG. 11 is a schematic diagram illustrating an application example of an area to be under a duty of care. FIG. 11 illustrates, for example, an example where an area to beware of pedestrians E6 has been set on a road having a school zone road sign Rs2. As illustrated in FIG. 11, a range prescribed by the school zone road sign Rs2 and a range within a predetermined distance, for example, five meters, from the school zone road sign RsS2 toward this side are set in the area to beware of pedestrians E6.

The output control function is applicable to areas to be under a duty of care other than the areas to be under a duty of care described above by use of FIG. 7 to FIG. 11. For example, the output control function is applicable to a case where those on the left are prioritized at an intersection without a traffic light, a case where a vehicle overtakes a vehicle in the front (a parked vehicle) by going over a lane, a case where a vehicle lets a motorcycle behind to the left go first when making a left turn, or a case where a vehicle crosses a sidewalk of a convenience store, for example.

With respect to the above described embodiment, the example where an area to be under a duty of care is identified on the basis a road sign on a signboard has been described, but an area to be under a duty of care may be identified on the basis of a painted road sign.

### (3) Application Example of Travel Information

With respect to the above described embodiment, the example where driving behavior data is used as travel information has been described, the driving behavior data being represented by pairs of variance values of acceleration in traveling directions obtained in areas to be under a duty of care and average velocities in the traveling directions obtained in the areas to be under a duty of care, but not necessarily two items are included.

For example, the output control unit 17 may control whether or not to output information related to fatigue in a driver, on the basis of an increasing trend of a second distribution of variance values of acceleration accumulated by the accumulating unit 13 after a first distribution of variance values of acceleration accumulated by the accumulating unit, the increasing trend being in relation to the first distribution.

More specifically, the output control unit 17 calculates a representative value of the first distribution by executing predetermined statistical processing of the variance values of acceleration included in the first distribution, for example, calculation of the average, the median, or the mode. Furthermore, the output control unit 17 calculates a representative value of the second distribution by executing statistical processing of the variance values of acceleration included in the second distribution, the statistical processing being similar to the statistical processing applied to the first distribution. Thereafter, the output control unit 17 determines whether or not the representative value of the second distribution is equal to or larger than a threshold Th2 set on the basis of the representative value of the first distribution. A value larger than the representative value of the first distribution, for example, the representative value of the first distribution + a margin β, may be set as this threshold Th2. In a case where the representative value of the second distribution is equal to or larger than the threshold Th2, the degree of buildup of fatigue is able to be presumed to have increased. In this case, the output control unit 17 outputs information related to fatigue in the driver. Even in a case where there are individual differences among degrees of buildup of fatigue in drivers, mental fatigue caused by provision of information to a driver who is not fatigued and omission in provision of information to a driver who is fatigued are able to be reduced.

Furthermore, with respect to the above described embodiment, the example where an increasing trend is determined by use of a distribution of variance values of acceleration has been described, but the distribution of variance values of acceleration is not necessarily used. For example, the output control unit 17 may output information related to fatigue in a driver on the basis of changes in variance values of acceleration accumulated by the accumulating unit 13 during travel in areas to be under a duty of care. By way of example only, in a case where a variance value of acceleration in an area to be under a duty of care is newly accumulated by the accumulating unit 13, the output control unit 17 determines whether or not the frequency at which a variance value of acceleration equal to or larger than a threshold Th3 is observed in a predetermined time period from and earlier than a time point at which the variance value is newly accumulated is equal to or larger than a threshold Th4. In a case where the frequency at which a variance value of acceleration equal to or larger than the threshold Th3 is observed in the predetermined time period is equal to or larger than the threshold Th4, it is able to be presumed that the degree of buildup of fatigue has increased. In this case, the output control unit 17 outputs information related to fatigue in the driver. Discontinuous driving behaviors leading to fatigue are thereby able to be evaluated. Therefore, accuracy of information related to fatigue is able to be improved effectively.

### (4) Notification of Recovery from Fatigue

With respect to the above described embodiment, the example where the degree of buildup of fatigue is determined has been described, but the degree of recovery from fatigue may be determined. By way of example only, in a case where a variance value of acceleration in an area to be under a duty of care is newly accumulated by the accumulating unit 13, the output control unit 17 may determine whether or not the frequency at which a variance value of acceleration less than a threshold Th5 is observed in a predetermined time period from and earlier than a time point at which the variance value is newly accumulated is equal to or larger than a threshold Th6. In a case where the frequency at which a variance value of acceleration less than the threshold Th5 is observed in the predetermined time period is equal to or larger than the threshold Th6, it is able to be presumed that the degree of buildup of fatigue has decreased, in other words, recovery from fatigue has been achieved. In another example, the output control unit 17 determines whether or not a representative value of a second distribution is equal to or smaller than a representative value of a first distribution. In a case where the representative value of the second distribution is equal to or smaller than the representative value of the first distribution, it is able to be presumed that the degree of buildup of fatigue has decreased, in other words, recovery from fatigue has been achieved. In yet another example, in a case where the intercept b2 of the second approximate straight line L2 is equal to or less than the intercept b1 of the first approximate straight line L1, the output control unit 17 may presume that the degree of buildup of fatigue has decreased, in other words, recovery from fatigue has been achieved. In this case, the output control unit 17 outputs information related to recovery of the driver from fatigue, for example, a message for notification of the recovery from fatigue.

### (5) Application Example of First Distribution

With respect to the above described embodiment, the example where a distribution of driving behavior data accumulated first since a time point at which the engine of a vehicle is started is selected as a first distribution has been described, but the embodiment is not limited to this example. For example, a distribution of driving behavior data corresponding to an approximate straight line having the smallest intercept among plural approximate straight lines respectively approximating plural distributions of driving behavior data of the travel information history 5 stored in the storage unit 4 may be selected as a first distribution. A first approximate straight line that tends to represent driving behaviors different from a second approximate straight line is thereby able to be used as a target of comparison for determination of an increase in the degree of buildup of fatigue.

Furthermore, in the above described embodiment, a distribution of driving behavior data accumulated immediately before accumulation of a second distribution of driving behavior data may be referred to as a first distribution. The degree of buildup of fatigue or the degree of recovery from fatigue is thereby able to be evaluated, with the latest changes in the driving behaviors finely reflected.

### (6) Application Example of Notification of Information

For example, in a case where the notification 2B illustrated in FIG. 2 is performed, a gap between the intercept b1 of the first approximate straight line L1 and the intercept b2 of the second approximate straight line L2, for example, the absolute value or square value of the difference, may be calculated as an increase of points indicating the level of fatigue or a decrease of points indicating the level of fatigue, in other words, points indicating recovery from fatigue. By way of example only, in a case where the sign of a subtracted value resulting from subtraction of the intercept b1 from the intercept b2 is positive, the output control unit 17 displays or outputs by sound the increase of points indicating the level of fatigue resulting from normalization of the subtracted value to a predetermined numerical range, for example, a range of 0 to 10. In another example, in a case where the sign of the subtracted value resulting from subtraction of the intercept b1 from the intercept b2 is negative, the output control unit 17 displays or output by sound the decrease of points indicating the level of fatigue resulting from normalization of the subtracted value to a predetermined numerical range, for example, a range of 0 to 10, in other words, points indicating recovery from fatigue. In yet another example, the output control unit 17 may output by display or output by sound changes in the increase of points indicating the level of fatigue calculated chronologically and/or the decrease of points indicating the level of fatigue.

### (7) Fatigue + Stress

As described above, stress does not necessarily lead to fatigue, but may cause some mental damage. Accordingly, the information processing apparatus 1 may correct a variance value of acceleration obtained as travel information, according to the level of stress. By way of example only, in correcting the variance value of acceleration, the information processing apparatus 1 may use at least one of the scales and frequency of potential accident cases.

The scales of potential accident cases refer to magnitudes of gravitational acceleration having negative values, that is, magnitudes of acceleration acting vertically upward. For example, the larger the acceleration acting vertically upward is, the larger the correction coefficient by which the variance value of acceleration is multiplied is made, and the smaller the acceleration acting vertically upward, the larger the correction coefficient by which the variance value of acceleration is multiplied is made. By way of example only, in a case where the acceleration acting vertically upward is equal to or larger than 0, the variance value of acceleration is multiplied by a correction coefficient equal to or larger than 1, and in a case where the acceleration acting vertically upward is less than 0, the variance value of acceleration is multiplied by a correction coefficient equal to or larger than 0 and less than 1. Furthermore, the higher the frequency of potential accident cases is, the larger the correction coefficient by which the variance value of acceleration is multiplied is made, and the lower the frequency of potential accident cases is, the larger the correction coefficient by which the variance value of acceleration is multiplied is made. By way of example only, in a case where the frequency of potential accident cases is equal to or larger than a threshold Th7, the variance value of acceleration is multiplied by a correction coefficient equal to or larger than 1, and in a case where the frequency of potential accident cases is less than the threshold Th7, the variance value of acceleration is multiplied by a correction coefficient equal to or larger than 0 and less than 1.

In addition, in a case where the positional information on the information processing apparatus 1 corresponds to a highly risky situation, such as the nighttime, the school commuting time, or the road width of the road travelled, the variance value of acceleration may be multiplied by a correction coefficient equal to or larger than 1. On the contrary, in a case where the positional information on the information processing apparatus 1 does not correspond to the highly risky situation, such as the night time, the school commuting time, or the road width of the road travelled, the variance value of acceleration may be multiplied by a correction coefficient equal to or larger than 0 and less than 1.

### (8) Client Server System

With respect to the above described embodiment, the example where the information processing apparatus 1 provides the output control function has been described, but the output control function may be provided as an output control service by a client server system.

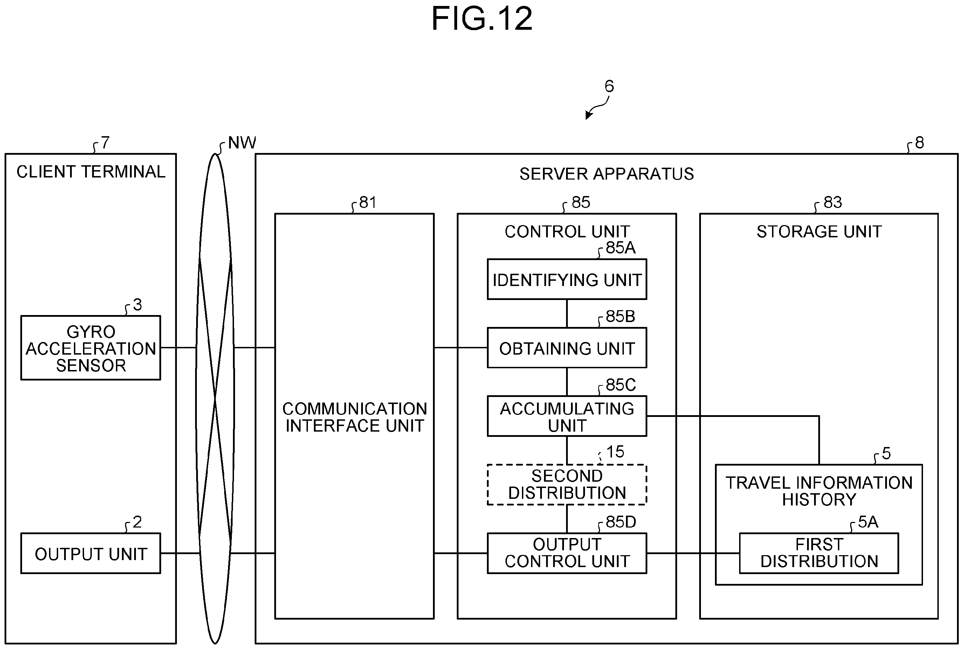

FIG. 12 is a diagram illustrating an example of a functional configuration of a client server system according to an application example. In one aspect, the output control service is provided from a server apparatus 8 to a client terminal 7, in a client server system 6 illustrated in FIG. 12. Functional units having the same functions as the functional units illustrated in FIG. 1 are assigned with the same reference signs and a detailed description of the functional units assigned with the same reference signs will be omitted.

As illustrated in FIG. 12, the client server system 6 may include the client terminal 7 and the server apparatus 8. The client terminal 7 and the server apparatus 8 are communicably connected via a network NW. For example, the network NW may be wired or wireless, and may be a communication network of any type, such as the Internet or a local area network (LAN).

FIG. 12 illustrates, for convenience of explanation, the example where one client terminal 7 is connected to one server apparatus 8, but a single client terminal 7 may be connected to a single server apparatus 8, of course.

The client terminal 7 is an example of a terminal device that receives the output control service provided. The client terminal 7 may be implemented by any computer, and by way of example only, may be implemented by the information processing apparatus 1 illustrated in FIG. 1.

Because the output control service is provided by the server apparatus 8, the client terminal 7 may be not provided with the identifying unit 11, the obtaining unit 12, the accumulating unit 13, and the output control unit 17 illustrated in FIG. 1. Instead, the client terminal 7 has an output unit 2 that has functions similar to those of the information processing apparatus illustrated in FIG. 1 to output information related to fatigue in a driver. Furthermore, the client terminal 7 has a gyro acceleration sensor 3 having functions similar to those of the information processing apparatus 1 illustrated in FIG. 1 to upload travel information to the server apparatus 8.

When the server apparatus 8 performs the above described identification of passage through an area to be under a duty of care, the identification may be performed by any method, but in a case where map matching is used, a positional information measuring unit may be included for positional information to be uploaded. Furthermore, in a case where image recognition is adopted, an imaging unit may be included to upload an image.

The server apparatus 8, on the other hand, is an example of a computer that provides the output control service. By way of example only, the server apparatus 8 may be implemented as a server that provides the output control service on premises. In addition, the server apparatus 8 may provide the output control service as a cloud service by being implemented as an application of Software as a Service (SaaS) type.

A functional configuration of the server apparatus 8 that provides the output control service will be described. FIG. 12 is a schematic of blocks corresponding to functions that the server apparatus 8 has. As illustrated in FIG. 8, the server apparatus 8 has a communication interface unit 81, a storage unit 83, and a control unit 85. FIG. 1 merely illustrates excerpts corresponding to functional units related to the output control service, and the server apparatus 8 may include any other functional unit not illustrated in the figure, for example, any functional unit to be included in an existing computer by default or optionally.

The communication interface unit 81 corresponds to an example of a communication control unit that controls communication with another device, for example, the client terminal 7. By way of example only, the communication interface unit 81 is implemented by a network interface card, such as a LAN card. For example, the communication interface unit 81 may receive positional information and/or an image, in addition to travel information, from the client terminal 7. Furthermore, the communication interface unit 81 may output, for example, information related to fatigue in a driver, to the client terminal 7.

The storage unit 83 is a functional unit to store various kinds of data. By way of example only, the storage unit 18 is implemented by an internal, external, or auxiliary storage of the server apparatus 8. For example, the storage unit 83 stores a travel information history 5 that may correspond to the travel information history 5 illustrated in FIG. 1.

The control unit 85 is a processing unit that executes overall control of the server apparatus 8. For example, the control unit 85 is implemented by a hardware processor. As illustrated in FIG. 12, the control unit 85 has an identifying unit 85A, an obtaining unit 85B, an accumulating unit 85C, and an output control unit 85D.

These identifying unit 85A, obtaining unit 85B, accumulating unit 85C, and output control unit 85D may correspond to the identifying unit 11, obtaining unit 12, accumulating unit 13, and output control unit 17 that are illustrated in FIG. 1. For example, except for the following three points, functions of the identifying unit 85A, the obtaining unit 85B, the accumulating unit 85C, and the output control unit 85D may be not different from the functions of the identifying unit 11, the obtaining unit 12, the accumulating unit 13, and the output control unit 17. The first point is that the identifying unit 85A may obtain sensor information, such as positional information and/or an image, via the network NW. The second point is that the obtaining unit 85B may obtain travel information via the network NW. The third point is that the output control unit 85D may transmit information related to fatigue in a driver via the network NW.

Similarly to the information processing apparatus 1 illustrated in FIG. 1, the client server system 6 according to this application example configured as described above enables improvement in accuracy of information related to fatigue.

### System

The processing procedures, control procedures, specific names, and information including various kinds of data and parameters, which are described in this document and illustrated in the drawings, may be modified in any way unless specified otherwise.

Furthermore, the components of each apparatus in the drawings have been illustrated functionally and conceptually, and are not necessarily configured physically as illustrated in the drawings. That is, specific modes of separation and integration of each apparatus are not limited to those illustrated in the drawings. All or a part of each apparatus may thus be functionally or physically separated or integrated in any units according to various loads and use situations.

Furthermore, all or any part of the processing functions executed at each apparatus may be implemented by a central processing unit (CPU) and a program analyzed and executed by the CPU, or may be implemented as hardware by wired logic.

### Hardware

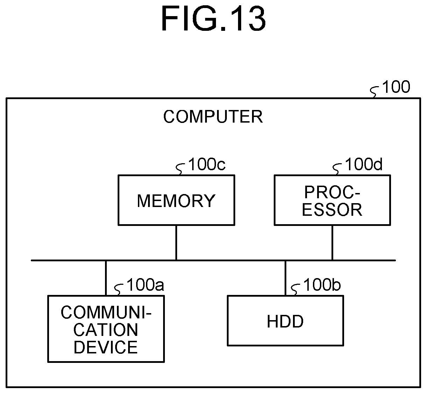

An example of a hardware configuration of a computer that executes an information processing program having the same functions as the information processing apparatus described with respect to the embodiment will be described next. FIG. 13 is a diagram for explanation of the example of the hardware configuration. As illustrated in FIG. 13, a computer 100 has a communication device 100a, a hard disk drive (HDD) 100b, a memory 100c, and a processor 100d. Furthermore, the units illustrated in FIG. 13 are connected to one another via a bus, for example.

The communication device 100a is a network interface card, for example, and performs communication with another server. The HDD 100b stores therein a program that causes the functions illustrated in FIG. 1 or FIG. 12, for example, to operate, and a database (DB).

The processor 100d causes a process to operate by: reading the program that executes the same processing as the processing units illustrated in FIG. 1 or FIG. 12, for example; and loading the program into the memory 100c, the process executing the functions described by reference to FIG. 1 or FIG. 12, for example. For example, the process executes the same functions as the processing units that the computer 100 has. Specifically, the processor 100d reads a program having the same functions as the processing units including the identifying unit 11, the obtaining unit 12, the accumulating unit 13, and the output control unit 17, from the HDD 100b, for example. The processor 100d then executes a process executing processing similar to that of the identifying unit 11, the obtaining unit 12, the accumulating unit 13, and the output control unit 17, for example.

As described above, by reading and executing a program, the computer 100 operates as an information processing apparatus that executes various processing methods. Furthermore, by reading the program from a recording medium by means of a medium reading device and executing the program read, the computer 100 is also capable of implementing the same functions as the above described embodiment. The program referred to in this other embodiment is not necessarily executed by the computer 100. For example, in a case where another computer or server executes the program, or in a case where the other computer and server execute the program in cooperation with each other, the functions that the information processing apparatus described with respect to the embodiment has may be implemented similarly.

This program may be distributed via a network, such as the Internet. Furthermore, the program may be executed by being recorded in a computer-readable recording medium, such as a hard disk, a flexible disk (FD), a CD-ROM, a magneto-optical disk (MO), or a digital versatile disc (DVD), and being read by the computer from the recording medium.

### Reference Signs List

- 1: INFORMATION PROCESSING APPARATUS
- 2: OUTPUT UNIT
- 3: GYRO ACCELERATION SENSOR
- 4: STORAGE UNIT
- 5: TRAVEL INFORMATION HISTORY
- 5A: FIRST DISTRIBUTION
- 10: CONTROL UNIT
- 11: IDENTIFYING UNIT
- 12: OBTAINING UNIT
- 13: ACCUMULATING UNIT
- 15: SECOND DISTRIBUTION
- 17: OUTPUT CONTROL UNIT

## Claims

1. An information processing apparatus, including:
an identifying means that identifies places where a driver of a vehicle is under a duty of care;
an obtaining means that obtains travel information on the vehicle at the places identified by the identifying means;
an accumulating means that accumulates travel information on the vehicle, the travel information having been obtained by the obtaining means; and
an output control means that outputs information related to fatigue in the driver, on the basis of the travel information on the vehicle accumulated by the accumulating means.

2. The information processing apparatus according to claim 1, wherein
the accumulating means accumulates variance values of acceleration in traveling directions, the variance values being obtained as the travel information on the vehicle respectively for the places by the obtaining means, and
the output control means outputs the information related to the fatigue in the driver, on the basis of a change in the variance values of the acceleration accumulated by the accumulating means during travel through the places.

3. The information processing apparatus according to claim 2, wherein the output control means controls whether or not to output the information related to the fatigue in the driver, on the basis of an increasing trend of a second distribution in relation to a first distribution of variance values of acceleration accumulated by the accumulating means, the second distribution being a distribution of variance values of acceleration accumulated after the first distribution by the accumulating means.

4. The information processing apparatus according to claim 2 or 3, wherein
the accumulating means accumulates data represented by pairs of: the variance values of the acceleration in the traveling directions; and average velocities at the places or scales of intersections at the places, and
the output control means controls whether or not to output the information related to the fatigue in the driver, on the basis of an increasing trend of a second approximate straight line in relation to a first approximate straight line approximating a first distribution of the data accumulated by the accumulating means, the second approximate straight line approximating a second distribution of the data accumulated by the accumulating means.

5. The information processing apparatus according to claim 4, wherein the output control means controls whether or not to output the information related to the fatigue in the driver, on the basis of whether or not an intercept of the second approximate straight line is equal to or larger than a threshold determined by an intercept of the first approximate straight line.

6. The information processing apparatus according to claim 4 or 5, wherein the output control means selects, as the first distribution, a distribution of data accumulated first by the accumulating means since a time point at which an engine of the vehicle is started, or a distribution of data corresponding to an approximate straight line having the smallest intercept among plural approximate straight lines respectively approximating plural distributions of the data accumulated by the accumulating means.

7. The information processing apparatus according to any one of claims 1 to 6, wherein
the accumulating means accumulates variance values of acceleration in traveling directions, the variance values being obtained as the travel information on the vehicle respectively for the places by the obtaining means, and
the output control means controls whether or not to output the information related to the fatigue in the driver, on the basis of a frequency at which a variance value of the acceleration exceeds a threshold.

8. The information processing apparatus according to any one of claims 1 to 7, wherein
the identifying means identifies a stop area,
the obtaining means obtains travel information on the vehicle in the stop area identified by the identifying means,
the accumulating means accumulates the travel information in the stop area, the travel information having been obtained by the obtaining means, and
the output control means executes the information related to the fatigue in the driver, on the basis of the travel information for the stop area, the travel information having been accumulated by the accumulating means.

9. The information processing apparatus according to any one of claims 1 to 8, wherein the output control means outputs, as the information related to the fatigue in the driver, a degree of increase in the fatigue in the driver, changes in the degree of increase, an alert for the driver and related to the fatigue, or a statistical value of times each taken from a time point at which an engine of the vehicle is started to output of the alert.

10. An output control method which is implemented by an information processing apparatus and with which the information processing apparatus executes a process including:
identifying a place where a driver of a vehicle is under a duty of care;
obtaining travel information on the vehicle at the place identified;
accumulating the obtained travel information on the vehicle; and
outputting information related to fatigue in the driver, on the basis of the accumulated travel information on the vehicle.

11. An output control program that causes a computer to execute a process including:
identifying a place where a driver of a vehicle is under a duty of care;
obtaining travel information on the vehicle at the place identified;
accumulating the obtained travel information on the vehicle; and
outputting information related to fatigue in the driver, on the basis of the accumulated travel information on the vehicle.
